(19) Europäisches Patentamt
European Patent Office
Office européen des brevets

(11) Veröffentlichungsnummer: **0 029 115**
**B1**

(12) # EUROPÄISCHE PATENTSCHRIFT

(45) Veröffentlichungstag der Patentschrift: **12.09.84**

(21) Anmeldenummer: **80106150.8**

(22) Anmeldetag: **10.10.80**

(51) Int. Cl.³: **C 07 D 471/22,** A 61 K 31/475 // (C07D471/22, 221/00, 221/00, 221/00, 209/00)

(54) **N(b)-quartäre Derivate von 10-Bromajmalin und 10-Bromisoajmalin, Verfahren und Zwischenprodukte zur Herstellung der Derivate und die Derivate enthaltende Arzneimittel.**

(30) Priorität: **13.10.79 DE 2941529**

(43) Veröffentlichungstag der Anmeldung: **27.05.81 Patentblatt 81/21**

(45) Bekanntmachung des Hinweises auf die Patenterteilung: **12.09.84 Patentblatt 84/37**

(84) Benannte Vertragsstaaten: **AT BE CH DE FR GB IT LI LU NL SE**

(56) Entgegenhaltungen:
**DE - A - 2 025 286**
**DE - B - 1 196 207**
**DE - C - 1 154 120**

**Chemical Abstracts, Band 79, Nr. 9, 3. September 1973, Columbus, Ohio, USA V. AHMAD et al. "Ajmaline series" Seite 394, Spalte 1, Abstract Nr. 53652b**

**Die Akte enthält technische Angaben, die nach dem Eingang der Anmeldung eingereicht wurden und die nicht in dieser Patentschrift enthalten sind.**

(73) Patentinhaber: **Kali-Chemie Pharma GmbH, Hans-Böckler-Allee 20 Postfach 220, D-3000 Hannover 1 (DE)**

(72) Erfinder: **Kehrbach, Wolfgang Dipl.-Chem. Dr.rer.nat., Altenbekener Damm 41, D-3000 Hannover 1 (DE)**
Erfinder: **Wegener, Joachim Dipl.-Chem. Dr.rer.nat., Mühlenstrasse 41, D-3201 Algermissen (DE)**
Erfinder: **Kühl, Ulrich, Dr.med.vet., Bergener Strasse 9, D-3000 Hannover 61 (DE)**
Erfinder: **Budden, Renke Dr. med.vet., Rodewaldstrasse 18, D-3000 Hannover (DE)**
Erfinder: **Buschmann, Gerd, Dr.med.vet., Matthäikirchstrasse 27, D-3000 Hannover 81 (DE)**

(74) Vertreter: **Lauer, Dieter, Dr., c/o Kali-Chemie Aktiengesellschaft Postfach 220, D-3000 Hannover 1 (DE)**

EP 0 029 115 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft $N_b$-quartäre Derivate von 10-Bromajmalin und 10-Bromisoajmalin, Verfahren und Zwischenprodukte zur Herstellung der Derivate und die Derivate enthaltende Arzneimittel.

Es ist bekannt (DE-C-1 154 120, DE-C-1 196 207, DE-C-1 620 559, DE-A-2 025 286), dass sich $N_b$-quartäre Derivate von Ajmalin und Isoajmalin durch wertvolle pharmakologische, insbesondere antiarrhythmische Wirkungen auszeichnen. Ein unter dem Warenzeichen Neo-Gilurytmal im Handel befindliches Präparat enthält als Wirksubstanz einen Vertreter dieser bekannten Derivate, das $N_b$-Propyl-Ajmalinium-Hydrogentartrat. Unter dem Warenzeichen Tachmalcor ist ein weiterer Vertreter dieser Derivate, das $N_b$-(2-Hydroxy-3-diäthylamino-)propyl-ajmalinium Hydrogentartrat bekannt geworden.

Bekannt ist auch das 10-Bromajmalin (Anet et al., J. Chem. Soc. 1954, Part I, 1242). Über dessen antiarrhythmische Wirkungen finden sich in der Literatur widersprechende Aussagen. So soll laut Ahmad et al., Pakistan J. Sci. Ind. Res., Vol. 15, Nr. 4–5 (1972), S. 246–251, 10-Bromajmalin dem Ajmalin überlegen sein, während A. Petter et al., Naunyn-Schmiedeberg's Arch. exp. Path. und Pharmak. 243 (1962), 519, dem 10-Bromajmalin bei gleicher Dosierung eine geringere Wirkung als Ajmalin zuschreiben und E. Völkner et al., Z. Ges. Exp. Med., 135 (1962), 350, berichten, dass 19-Bromajmalin schneller als Ajmalin zu AV-Blockierungen führt.

Der Erfindung liegt die Aufgabe zugrunde, bessere Wirkstoffe als die bekannten $N_b$-quartären Ajmalin- und Isoajmalin-Derivate bereitzustellen.

Die Lösung dieser Aufgabe gelingt durch Bereitstellung von $N_b$-quartären 19-Bromajmalin- und 10-Bromisoajmalin-Derivaten der allgemeinen Formel I

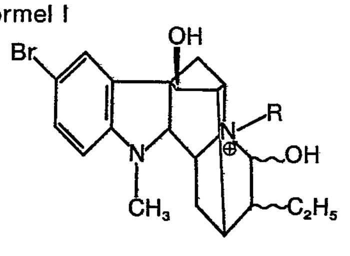

$A^{\ominus}$ (I)

in der

A das Anion einer anorganischen oder organischen Säure und

R ein bis zu 10 Kohlenstoffatome und gegebenenfalls Halogen oder Sauerstoff oder Stickstoff oder Sauerstoff und Stickstoff enthaltender Rest ist,

wobei

a) R ein Rest der allgemeinen Formel IIa

$-(CH_2)_n-CH-CH_2-X$ (IIa)
|
Y

oder

b) R ein Rest der allgemeinen Formel IIb

$-CH_2-CH-CH_2-X$ (IIb)
|
OH

ist, in denen

n den Wert 0 oder 1 annehmen kann

X Wasserstoff, gerad- oder verzweigtkettiges Alkyl, gegebenenfalls substituiertes Phenyl, Hydroxy, Dialkylamino, Pyrrolidino, Piperidino oder Morpholino und

Y Wasserstoff oder Methyl bedeuten

oder

c) R ein Methyl-, Allyl-, Cyclopropylmethyl-, Benzyl-, 4-Fluorbenzyl-, 4-Methoxybenzyl- oder 2-Hydroxy-2-phenyl-äthyl-Rest ist.

Der Rest R ist insbesondere ein Methyl-, Äthyl-, Allyl-, Propyl-, Butyl-, 3-Methyl-butyl-, Hexyl-, Decyl-, 2-Hydroxy-äthyl-, 2-Diäthylamino-äthyl-, 3-Diäthylamino-propyl-, 2-(1-Pyrrolidinyl)-äthyl-, 2-(1-Piperidinyl)-äthyl-, 2-(4-Morpholinyl)-äthyl- oder 2-Hydroxy-3-(1-piperidinyl)-propyl-Rest.

Vorteilhafterweise ist A das Anion einer pharmakologisch annehmbaren Säure, vorzugsweise das Anion der Weinsäure, Oxalsäure, Citronensäure, Salzsäure oder Phosphorsäure, insbesondere dasAnion der Weinsäure.

Die neuen Derivate können auf verschiedene Weise hergestellt werden. In einem Fall wird 10-Bromajmalin der Formel III-n

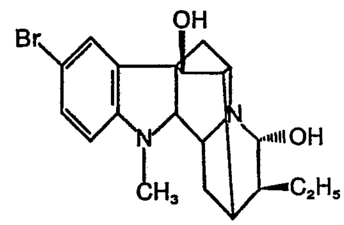

(III–n)

in der die 21-Hydroxy-Gruppe α-ständig und die 20-Äthyl-Gruppe β-ständig ist, oder 10-Bromisoajmalin der Formel III-i

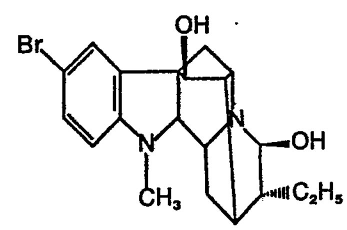

(III–i)

in der die 21-Hydroxy-Gruppe β-ständig und die 20-Äthyl-Gruppe α-ständig ist, entweder mit einer bis zu 10 Kohlenstoffatome enthaltenden Verbindung der Formeln Va oder Vb

$Z-(CH_2)_n-CH-CH_2-X$ (Va)
|
Y

$Z-CH_2-CH-CH_2-X$ (Vb)
|
OH

in denen, n, X und Y die oben genannte Bedeutung haben und Z Chlor, Brom, Jod oder die Tosylgruppe bedeutet, oder mit

Methyl-, Allyl-, Cyclopropylmethyl-, Benzyl-, 4-Fluorbenzyl-, 4-Methoxybenzyl-Chlorid, Bromid, Jodid, Oder Tosylat umgesetzt.

Die Ausgangsstoffe können dabei in äquimolaren Mengen eingesetzt werden. Vorteilhafterweise wird ein Überschuss des «Alkylierungsmittels» verwendet. Die Umsetzung erfolgt vorzugsweise

in einem Lösungsmittel, das gegenüber den Reaktionsteilnehmern inert ist. Geeignete Lösungsmittel sind beispielsweise Acetonitril, Chloroform, Dimethylformamid, Sulfolan, Dioxan oder Alkohole, wie z.B. Methanol oder Äthanol. Soweit das «Alkylierungsmittel» unter den Reaktionsbedingungen nicht mit sich selbst reagiert, kann auch das «Alkylierungsmittel» als Lösungsmittel dienen. Vorteilhafterweise führt man die Umsetzung bei der Siedetemperatur des Lösungsmittels aus, die Umsetzung kann aber insbesondere bei den höher siedenden Lösungsmitteln auch unterhalb der Siedetemperatur ausgeführt werden.

Die aus der vorerwähnten Umsetzung resultierenden quartären Salze von 10-Bromajmalin- oder 10-Bromisoajmalin können bereits Endprodukte im Sinne der Formel I sein, beispielsweise wenn das quartäre Salz für die beabsichtigte galenische Zubereitung geeignet oder das Anion des quartären Salzes ein pharmakologisch annehmbares Anion ist.

Wenn das quartäre Salz für die beabsichtigte Verwendung nicht geeignet ist, beispielsweise weil das Salz hygroskopisch oder das Anion pharmakologisch nicht annehmbar ist, werden die erhaltenen quartären Salze durch Behandlung mit Alkalien in die ringoffenen Aldehydbasen der Formel IV

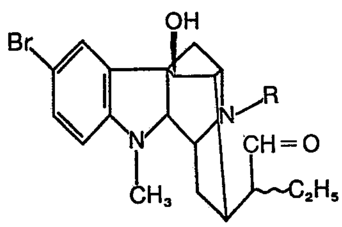

(IV)

in der R die vorstehend genannte Bedeutung hat, umgewandelt und diese durch Umsetzung mit einer Säure HA, in der A die vorstehend genannte Bedeutung hat, in das quartäre Derivat der Formel I überführt.

Als zur Umwandlung der quartären Salze in die ringoffene Aldehydbase geeignete Alkalien kommen wässrige Laugen, wie z.B. 10%ige Natrolauge oder wässrige Natriumhydrogencarbonat- oder Natriumcarbonat-Lösungen in Betracht. Zweckmässigerweise wird in Gegenwart eines geeigneten Extraktionsmittels gearbeitet. Geeignet sind alle inerten wassernichtmischbaren Lösungsmittel, wie Chloroform, Methylenchlorid, Essigester oder Diäthyläther, in denen die Basen eine ausreichende Löslichkeit besitzen. Nach Abdestillation des Extraktionsmittels, vorteilhafterweise Vakuumdestillation, liegen die Basen in amorpher Form vor. Für die Weiterverarbeitung in das quartäre Derivat ist es nicht unbedingt erforderlich, die ringoffene Aldehydbase zu isolieren; es kann auch der getrocknete und geklärte Extrakt verwendet werden.

In einer anderen Verfahrungsvariante wird 10-Bromajmalin oder 10-Bromisoajmalin mit einem Epoxid der Formel Vb′

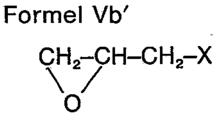

(Vb′)

in der X die oben genannte Bedeutung hat oder mit Epoxystyrol umgesetzt.

Im Fall der Umsetzung von 10-Bromajmalin oder 10-Bromisoajmalin mit Epoxiden fällt sofort die ringoffene Aldehydbase an, die man mit der Säure HA, in der A die vorstehend genannte Bedeutung hat, in das quartäre Derivat der Formel I überführt.

Da die ringoffenen Aldehydbasen in amorpher, d.h. nichtkristalliner Form vorliegen, besitzen sie keinen eindeutigen Schmelzpunkt. Auch der Drehwert scheidet für eine eindeutige Charakterisierung aus, da bei der Quarternisierung von Bromajmalin bzw. Bromisoajmalin eine Isomerisierung an den Zentren C-20 und C-21 auftreten kann. Diese Isomerisierung führt, gleichgültig ob man von reinem Bromajmalin oder reinem Bromisoajmalin ausgeht, zu einem Gemisch der stereoisomeren quartären Salze bzw. Aldehydbasen, wobei die Zusammensetzung des Gemisches jedoch unterschiedlich ist, je nach dem, ob man von Bromajmalin oder Bromisoajmalin ausgeht. Der Grad der Isomerisierung ist vom Raumanspruch des Restes R und der Art seiner Substitution abhängig. Das Isomerenverhältnis kann auch von Versuch zu Versuch schwanken, wenn die Reaktionsbedingungen nicht exakt übereinstimmen. Die Isomerisierung tritt nur in wenigen Fällen nicht auf. Bei der Überführung der ringoffenen Aldehydbasen in die quartären Derivate kann sich das Verhältnis der Isomeren weiter verändern.

Die ringoffenen Aldehydbasen lassen sich daher am vorteilhaftesten durch die Lage der Resonanz des aldehydischen Protons im $^1$H-Kernresonanzspektrum und durch das Auftreten einer Carbonyl-Bande im IR-Spektrum charakterisieren. Die Resonanz des aldehydischen Protons tritt bei 9,0–9,6 $\delta$ auf, wärend die Carbonylbande bei 1700–1720 $cm^{-1}$ erscheint. Als repräsentativ für die ringoffenen Aldehydbasen ist in Fig. 1 das IR-Spektrum der ringoffenen Aldehydbase von $N_b$-Propyl-10-brom-isoajmalin und in Fig. 2 ein Ausschnitt aus dem 90 MHz $^1$H-FT-NMR-Spektrum derselben Verbindung dargestellt. Im IR-Spektrum ist die C=O Valenzsschwingung bei 1710 $cm^{-1}$ zu erkennen. Im NMR-Spektrum ist die bei tiefem Feld auftretende Resonanz des O=CH-Aldehydprotons am C-21 charakteristisch, die durch Kopplung mit dem einzigen benachbarten Proton am C-20 mit einer Frequenz von 4 Hz aufgespalten ist. Das Auftreten von 2 Signalen ist auf das Vorliegen eines Isomerengemisches aus der n-Form (Signal bei 9,52) und der iso-Form (Signal bei 9,45) zurückzuführen. Aus der Höhe der jeweiligen Peaks kann auf das Isomeren-Verhältnis geschlossen werden.

Die in Substanz dargestellten oder im Extraktionsmittel gelöst vorliegenden ringoffenen Aldehydbasen werden anschliessend mit einer pharmakologisch oder physiologisch annehmbaren anorganischen oder organischen Säure umgesetzt, vorzugsweise mit Weinsäure, Oxalsäure, Citronensäure, Salzsäure oder Phosphorsäure und dabei in die quartären Derivate überführt.

Soweit vorstehend oder nachfolgend von n-oder iso-Form gesprochen wird, bezieht sich diese Aussage ausschliesslich auf die Konfiguration am C-20. Die 21-OH-Gruppe steht üblicherweise trans-ständig, kann aber auch cis-ständig sein.

Die erfindungsgemässen $N_b$-quartären Derivate von 10-Bromajmalin bzw. 10-Bromisoajmalin zeigen bei arzneilicher Verwendung adrenolytische und herzrhythmisierende Wirkungen. Überraschenderweise sind jedoch die neuen Verbindungen wesentlich stärker wirksam und physiologisch verträglicher als die bekannten Ajmalin-Derivate. So zeigen die neuen Derivate im Vergleich zu den bekannten Ajmalin-Derivaten schon bei einer wesentlich geringeren Dosis eine vergleichbare Wirkung. Darüber hinaus ist die unerwünschte negative Inotropie, wie sie bei den Ajamlin-Derivaten beobachtet wird, schwächer, und zwar bei gleichzeitiger grösserer therapeutischer Breite .

Die erfindungsgemäss hergestellten Wirkstoffe sind sowohl in wässriger Lösung als auch in fester Form stabil und haltbar und können mit den üblichen Träger- oder Hilfsstoffen in arzneilich geeigneten Darreichungsformen therapeutisch angewendet werden.

Die Überlegenheit der erfindungsgemässen Wirkstoffe ergibt sich aus dem Vergleich der in der nachstehenden Tabelle für repräsentative Vertreter angegebenen pharmakologischen Daten mit den entsprechenden Daten des bekannten $N_b$-Propyl-Ajmalinium-Hydrogentartrats (Neo-Gilurytmal®).

In der Tabelle ist im einzelnen aufgeführt:

Die akute Toxizität bei oraler (p.o.) und intraperitonealer (i.p.) Applikation an männlichen NMRI-Mäusen der Gewichtsklasse 18 bis 22 g. Als $LD_{50}$ ist dabei diejenige Dosis in µmol/kg definiert, bei der die Mortalitätsrate am 7. Tag nach Applikation 50% der Versuchstiere beträgt. Die $LD_{50}$ wurde durch Probitanalyse [L. Cavalli-Sforza, Grundbegriffe der Biometrie, insbesondere der statistischen Methoden bei der Wertbemessung biologisch wirksamer Substanzen, Gustav Fischer Verlag, Stuttgart (1946)] berechnet.

Minimale Symptomdosis bei männlichen NMRI-Mäusen der Gewichtsklasse 18 bis 22 g gemäss dem Wirkungsbild nach Campbell und Richter [D.E.S. Campbell und W. Richter, Acta Pharmakol. Toxicol. 25 (1967), 345–363]: Als minimale Symptomdosis ist diejenige Dosis in µmol/kg definiert, bei welcher es bei 2 von 3 Mäusen nach i.p. Verabreichung zu Verhaltensänderungen kam. Die minimale Symptomdosis ist ein Mass für die unerwünschten Nebenwirkungen.

Verlängerung der funktionellen Refraktärzeit (fRZ.) und die Verminderung der Kontraktionskraft (Kraft) am isolierten linken Vorhof von weiblichen Albino-Pirbright-white-Meerschweinchen (MS) der Gewichtsklasse 300 bis 400 g gemäss der Doppelreizmethode von Govier [W.C. Govier, J. Pharmakol. Exp. Ther. 148 (1) (1965), 100–105]: Angegeben ist diejenige Konzentration in µmol/l, bei der es 18 Minuten nach Applikation zu einer Verlängerung der funktionellen Refraktärzeit auf 125% bzw. zu einer Verminderung der Kontraktionskraft auf 75% des Ausgangswertes kommt. Angegeben ist ausserdem der Quotient aus kontraktionskraftmindernder und refraktärzeitverlängernder Dosis. Dieser Quotient gibt Aufschluss über die «therapeutische Breite» der antiarrhythmischen Wirkung am isolierten Organ [K. Greef, Verh. Dtsch. Ges. Kreislaufforsch. 35 (1969) 88–97].

Der Einfluss der Wirksubstanz auf mit Aconitin infundierten männlichen Wista-Ratten der Gewichtsklasse 280 bis 350 g gemäss der Methode nach Raschak [M. Raschak, Arzneim.-Forsch. (Drug Res.) 25 (4) (1975), 639–641]: Angegeben ist die Differenz derjenigen Zeit in Δ%, bei der es im Vergleich zu einem Kontrollversuch, bei dem die Wirksubstanz durch das Vehikel (isotonische NaCl-Lösung oder Lösungsvermittler) ersetzt wurde, nach einer intravenösen Gabe an Wirksubstanz in einer Dosis von $^1/_{20}$ der an der Maus bestimmten i.p. $LD_{50}$ und anschliessender Infusion einer pro Zeiteinheit konstanten Aconitin-Dosis zum Auftreten von Rhythmusstörungen [Extrasystolen (ES), ventrikulärer Tachykardie (VT), Kammerflattern (KF)] kam.

Da einige der Daten in der Dimension µmol/kg bzw. µmol/l angegeben sind, ist in der Tabelle ausserdem noch das errechnete Molekulargewicht (MG) der Wirksubstanz angegeben.

Die folgenden Beispiele sollen den Gegenstand der Erfindung näher erläutern.

Die aus 10-Bromajmalin bzw. 10-Bromisoajmalin (Anet, Chakravarti, Robinson und Schlittler, J. Chem. Soc. 1954, Part I, 1242) gemäss den Beispielen hergestellten quartären Salze wurden nach Freisetzung der Aldehydbase mit L(+)-Weinsäure zu den Hydrogentartraten umgesetzt. Aldehydbasen mit stickstofffreiem Alkylrest wurden mit einer äquimolaren Weinsäuremenge, Aldehydbasen mit stickstoffhaltigem Alkylrest mit einer doppelt molaren Weinsäuremenge umgesetzt (ausgenommen Beispiele 10 und 22, wo mit einer äquimolaren Säuremenge umgesetzt wurde).

Allgemeine Arbeitsvorschrift für die Freisetzung der Aldehydbase

Das quartäre Salz (ca. 10 g) wird in 200–300 ml Wasser gelöst, unter Eiskühlung alkalisch gemacht und etwa fünfmal mit Essigsäureäthylester (siehe nachfolgende Variante A, B, D) oder Diäthyl-Äther (siehe nachfolgende Variante C) extrahiert.

Allgemeine Arbeitsvorschriften für die Herstellung der Hydrogentartrate

Variante A

Die getrocknete Essigsäureäthylesterlösung wird eingedampft, der Rückstand in wenig Methanol gelöst, die berechnete Menge L(+)-Weinsäure zugegeben und in ca. 1 l Essigsäureäthylester eingetropft. Das ausgefallene Hydrogentartrat wird abfiltriert.

Tabelle

| Wirksubstanz Beispiel | R | MG | $LD_{50}$ i.p. µmol/kg | p.o. µmol/kg | Min. Symptomdosis µmol/kg | isolierter MS-Vorhof Kraft µmol/l | fRZ µmol/l | Kraft/fRZ | Aconitin-Ratte ES Δ% | VT Δ% | KF Δ% |
|---|---|---|---|---|---|---|---|---|---|---|---|
| Vergleich (NeoGilurytmal) | n-$C_3H_7$ | 519 | 41 | 65 | 12 | 0,26 | 1,8 | 0,15 | +86 | +80 | +50 |
| 11.b) | $-CH_3$ | 569 | 190 | 1289 | 88 | 9,0 | 7,2 | 1,3 | +85 | +73 | +52 |
| 12.b) | $-C_2H_5$ | 583 | 122 | 1430 | 21 | 1,4 | 1,5 | 0,9 | +52 | +55 | +39 |
| 13.b) | n-$C_3H_7$ | 598 | 118 | 437 | 42 | 1,5 | 0,6 | 2,4 | +110 | +130 | +110 |
| 14.b) | n-$C_4H_9$ | 612 | 76 | 198 | 20 | 1,5 | 2,0 | 0,7 | +80 | +87 | +88 |
| 15.b) | n-$C_{10}H_{21}$ | 696 | 72 | >1440 | 287 | 113 | 24,8 | 4,6 | n.s. | n.s. | n.s. |
| 8.b) | $-(CH_2)_2-N$ 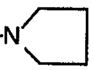 | 803 | 115 | 394 | 31 | 2,0 | 0,9 | 2,3 | +212 | >+149 | +129 |
| 9.b) | $-(CH_2)_2-N$ 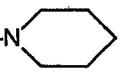 | 817 | 60 | 148 | 31 | 1,4 | 2,3 | 0,7 | +151 | +52 | +95 |
| 10 | $-(CH_2)_2-N$ 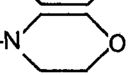 | 555 | 45 | 470 | 22 | 2,1 | 0,2 | 12,9 | +67 | +69 | +43 |
| 19.b) | $-(CH_2)_3-N(C_2H_5)_2$ | 819 | 122 | 686 | 31 | 2,0 | 4,4 | 0,5 | +220 | +180 | +158 |
| 16.b) | $-CH_2-$ 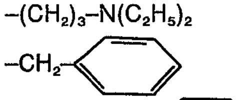 | 646 | 483 | 2277 | *) | 16,6 | 2,7 | 6,1 | +36 | +30 | +31 |
| 21 | $-CH_2-CHOH-$ 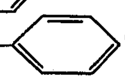 | 676 | 250 | 2175 | – | 3,2 | 3,8 | 0,8 | +89 | +92 | +72 |
| 22 | $-CH_2-CHOH-CH_2-N$ 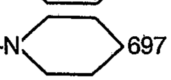 | 697 | 168 | 550 | 72 | 3,4 | 8,1 | 0,4 | +154 | +121 | +110 |

Erläuterungen:
n.s. = nicht signifikant
*) = Symptome erst im toxischen Bereich
– = nicht gemessen

Variante B

Die getrocknete Essigsäureäthylesterlösung wird auf ca. 100 ml eingedampft, durch Watte filtriert und in eine Lösung der berechneten L(+)-Weinsäuremenge in 100 ml Aceton eingetropft. Das ausgefallene Hydrogentartrat wird abfiltriert.

Variante C

Die Lösung der mit Diäthyl-Äther extrahierten Aldehydbase wird getrocknet und eingedampft. Der Rückstand wird in möglichst wenig Aceton aufgenommen und in die Lösung der berechneten L(+)-Weinsäuremenge in Aceton eingetropft. Das ausgefallene Hydrogentartrat wird abfiltriert.

Variante D

Die getrocknete Essigsäureäthylesterlösung wird auf ca. 100 ml eingedampft, durch Watte filtriert und in eine Lösung der berechneten Menge L(+)-Weinsäure eingetropft, die durch Verdünnen einer heiss gesättigten Weinsäurelösung in Aceton mit 1 l Essigsäureäthylester hergestellt worden war. Das ausgefallene Hydrogentartrat wird abfiltriert.

Wenn das Hydrogentartrat amorph anfällt, wird die Aldehydbase nochmals freigesetzt und das Hydrogentartrat – wie beschrieben – erneut gefällt.

Alle angegebenen Ausbeuten sind auf Bromajmalin bzw. Bromisoajmalin bezogen.

Alkylierung von 10-Bromajmalin

Beispiel 1

1.a) $N_b$-Methyl-10-brom-ajamlinium-jodid:

10 g 10-Bromajamalin und 8 ml Methyljodid wurden in 350 ml Acetonitril gelöst und 8 h unter Rückfluss erhitzt. Aus der abgekühlten Lösung kristallisierte $N_b$-Methyl-10-bromajmaliniumjodid.

Ausbeute: 9,5 g (70%); Fp.: 235–238 °C; reine n-Form.

1.b) $N_b$-Methyl-10-brom-ajmalinium-hydrogentartrat:

Die Herstellung erfolgte nach Variante A.

Ausbeute: 62%; Fp.: 150–155 °C; reine n-Form.

Beispiele 2–6

Die Herstellung der quartären Salze erfolgte analog Beispiel 1, durch Umsetzung von 10-Bromajmalin mit 1-Jodpropan (Beispiel 2) bzw. 1-Jodhexan (Beispiel 3) bzw. Allylbromid (Beispiel 4) bzw. 1-Jod-3-methyl-butan (Beispiel 5) bzw. 1-Bromdecan (Beispiel 6).

2.a) $N_b$-Propyl-10-brom-ajmalinium-jodid:

Ausbeute: 93%; Fp.: 273 °C dec; n: iso ca. 3:1.

2.b) N-$_b$Propyl-10-brom-ajmalinium-hydrogentartrat:

Die Herstellung erfolgte nach Variante B.

Ausbeute: 59%; Fp.: 180–185 °C; n:iso ca. 4:1.

3.a) $N_b$-Hexyl-10-brom-isoajmalinium-jodid:

Ausbeute: 53%; Fp.: 220–223 °C; n:iso ca. 1:4.

3.b) $N_b$-Hexyl-10-brom-isoajmalinium-hydrogentartrat:

Die Herstellung erfolgte nach Variante B.

Ausbeute: 45%; Fp.: 160–165 °C; n:iso ca. 2:3.

4.a) $N_b$-Allyl-10-brom-ajmalinium-bromid:

Ausbeute: 80%; Fp.: 245–248 °C; reine n-Form.

4.b) $N_b$-Allyl-10-brom-ajmalinium-hydrogentartrat:

Die Herstellung erfolgte nach Variante A.

Ausbeute: 41%; Fp.: 147–149 °C; reine n-Form.

5) $N_b$-(3-Methylbutyl)-10-brom-ajmalinium-jodid:

In Abweichung von Beispiel 1 wurde 14 Stunden unter Rückfluss erhitzt.

Ausbeute: 65%; Fp.: 240–245 °C; n:iso ca. 2:1.

6.a) $N_b$-Decyl-10-brom-ajmalinium-bromid:

In Abweichung von Beispiel 1 wurde 12 Stunden unter Rückfluss erhitzt.

Ausbeute: 34%; Fp.: 232 °C; n:iso ca. 7:3.

6.b) $N_b$-Decyl-10-brom-ajmalinium-hydrogentartrat:

Die Herstellung erfolgte nach Variante B.

Ausbeute: 21%; Fp.: 112–115 °C; n:iso ca. 1:1.

Beispiel 7

7.a) $N_b$-Diäthylaminoäthyl-10-brom-isoajmalinium-chlorid:

10 g 19-Bromajmalin wurden mit 5,72 g 2-Diäthylamino-äthylchlorid in 160 ml absolutem Dioxan versetzt und 9 h unter Rückfluss erhitzt. Nach dem Abkühlen kristallisierte das Produkt aus und wurde abgesaugt.

Ausbeute: 9,6 g (72%); Fp.: 211–213 °C.

7.b) $N_b$-Diäthylaminoäthyl-10-brom-isoajmalinium-bishydrogentartrat:

Die Herstellung des Bishydrogentartrates erfolgte nach Variante C.

Ausbeute: 52%; Fp.: 115–118 °C; n:iso ca. 1:4.

Beispiele 8–10

Die Herstellung der quartären Salze erfolgte analog Beispiel 7 durch Umsetzung von 10-Bromajmalin mit 1-Chlor-2-(1-Pyrrolidinyl)-äthan (Beispiel 8) bzw. 1-Chlor-2-(1-Piperidinyl)-äthan (Beispiel 9) bzw. 1-Chlor-2-(4-Morpholinyl)-äthan (Beispiel 10). Die Herstellung der Hydrogentartrate erfolgte wie im Beispiel 7 nach Variante C.

8.a) $N_b$-[2-(1-Pyrrolidinyl)-äthyl]-10-brom-isoajmaliniumchlorid:

Ausbeute: 48%; Fp.: 233–235 °C.

8.b) $N_b$-[2-(1-Pyrrolidinyl)-äthyl]-10-brom-isoajmalinium-bishydrogentartrat:

Ausbeute 31%; Fp.: 121–125 °C; n:iso ca. 1:6.

9.a) $N_b$-[2-(1-Piperidinyl)-äthyl]-10-brom-isoajmalinium-chlorid:

Ausbeute: 64%; Fp.: 234–236 °C.

9.b) $N_b$-[2-(1-Piperidinyl)-äthyl]-10-brom-isoajmalinium-bishydrogentartrat:

Ausbeute: 39%; Fp.: 124–127 °C; n:iso ca. 1:2.

10.a) $N_b$-[2-(4-Morpholinyl)-äthyl]-10-brom-ajmalinium-chlorid:

In Abweichung von Beispiel 7 wurde 48 Stunden unter Rückfluss erhitzt.

Ausbeute: 53%; Fp.: 225–229 °C.

10.b) $N_b$-[2-(Morpholinyl)-äthyl]-10-brom-isoajmalinium-chlorid:

5 g 10-Bromajmalin und 2,4 g 2-(4-Morpholinyläthyl)-chlorid wurden in 120 ml Äthanol gelöst und 48 Stunden unter Rückfluss erhitzt. Nach dem Einengen auf ca. 40 ml und Versetzen mit etwas Äther

kristallisierte das Produkt aus und wurde abgesaugt.

Ausbeute: 5,6 g (82%); Fp.: 245 °C dec; reine iso-Form.

Alkylierung von 10-Bromisoajmalin
Beispiele 11–15

Die Herstellung der quartären Salze erfolgte analog Beispiel 1 durch Umsetzung von 10-Bromisoajmalin mit Jodmethan (Beispiel 11) bzw. 1-Jodäthan (Beispiel 12) bzw. 1-Jodpropan (Beispiel 13) bzw. 1-Jodbutan (Beispiel 14) bzw. 1-Bromdecan (Beispiel 15). Die Herstellung der Hydrogentartrate erfolgte nach Variante D.

11.a) $N_b$-Methyl-10-brom-isoajmalinium-jodid:
Ausbeute: 76%; Fp.: 242–245 °C; reine iso-Form.

11.b) $N_b$-Methyl-10-brom-isoajmalinium-hydrogentartrat:
Ausbeute: 57%; Fp.: 128–132 °C; reine iso-Form.

12.a) $N_b$-Äthyl-10-brom-isoajmalinium-jodid:
Ausbeute: 85%; Fp.: 277–280 °C; reine iso-Form.

12.b) $N_b$-Äthyl-10-brom-isoajmalinium-hydrogentartrat:
Ausbeute: 72%; Fp.: 110–130 °C; reine iso-Form.

13.a) $N_b$-Propyl-10-brom-isoajmalinium-jodid:
Ausbeute: 86%; Fp.: 275 °C dec; n:iso ca. 1:6.

13.b) $N_b$-Propyl-10-brom-isoajmalinium-hydrogentartrat:
Ausbeute: 71%; Fp.: 100–120 °C; n:iso ca. 1:6.

14.a) $N_b$-Butyl-10-brom-isoajmalinium-jodid:
Ausbeute: 83%; Fp.: 265 °C dec; n:iso ca. 1:19.

14.b) $N_b$-Butyl-10-brom-isoajmalinium-hydrogentartrat:
Ausbeute: 53%; Fp.: 125–130 °C; n:iso ca. 1:10.

15.a) $N_b$-Decyl-10-brom-isoajmalinium-bromid:
Ausbeute: 55%; Fp.: 232 °C; n:iso ca. 1:3.

15.b) $N_b$-Decyl-10-brom-isoajmalinium-hydrogentartrat:
Ausbeute: 35%; Fp.: 110–115 °C; n:iso ca. 1:4.

Beispiel 16

16.a) $N_b$-Benzyl-10-brom-isoajmalinium-bromid:

10 g 10-Brom-isoajmalin wurden mit 10 ml Benzylbromid in 40 ml Chloroform 24 h bei Raumtemperatur gerührt. Nachdem bei Raumtemperatur unter Vakuum etwas Chloroform abgezogen wurde, wurde im Tiefkühlfach unter Zusatz von Aceton und Äther kristallisiert. Die Kristalle wurden aus Methanol/Äther umkristallisiert.

Ausbeute: 6 g (42%); Fp.: 225–227 °C; reine iso-Form.

16.b) $N_b$-Benzyl-10-brom-isoajmalinium-hydrogentartrat:

Die Herstellung erfolgte nach Variante D.

Ausbeute: 25%; Fp.: 130–132 °C; n:iso ca. 1:5.

Beispiel 17

$N_b$-(4-Fluorbenzyl)-10-brom-isoajmalinium-Bromid:

12 g 10-Brom-isoajmalin und 10 ml 4-Fluorbenzylbromid wurden in 100 ml Sulfolan gelöst und 1 h bei 80 °C unter Stickstoff gerührt. Der beim Verdünnen mit 200 ml Aceton ausgefallene Niederschlag wurde abfiltriert, mit Aceton gewaschen und aus Methanol/Äther umkristallisiert.

Ausbeute: 12,8 g (73%); Fp.: 247 °C; reine iso-Form.

Beispiel 18

$N_b$-(4-Methoxybenzyl)-10-brom-isoajmalinium-chlorid:

11 g 10-Brom-isoajmalin und 12 ml 4-Methoxybenzylchlorid wurden in 100 ml Sulfolan gelöst und 10 h bei 80 °C unter Stickstoff gerührt. Der ausgefallene Niederschlag wurde mit Aceton gewaschen und aus Methanol/Essigester umkristallisiert.

Ausbeute: 3,4 g (22%); Fp.: 228–232 °C; reine iso-Form.

Beispiel 19

19.a) $N_b$-Diäthylaminopropyl-10-brom-isoajmalinium-chlorid:

Die Herstellung erfolgte analog Beispiel 7 aus 10-Bromisoajmalin und mit Diäthylaminopropylchlorid als Alkylierungsmittel.

Fp.: 156 °C; n:iso ca. 1:1.

19.b) $N_b$-Diäthylaminopropyl-10-brom-isoajmalinium-bishydrogentartrat:

Der noch dioxanfeuchte Niederschlag des Chlorids wurde in wenig Wasser aufgenommen und zur Entfernung überschüssigen Alkylierungsmittels mit 100 ml Äther extrahiert. Nach Freisetzung der Aldehydbase wurde das Bishydrogentartrat nach Variante D hergestellt.

Ausbeute: 50%; Fp.: 118–120 °C; n:iso ca. 1:3.

Beispiel 20

$N_b$-(2-Hydroxyäthyl)-10-brom-isoajmalinium-chlorid:

15 g 10-Brom-isoajmalin und 50 ml Chloräthanol wurden in 100 ml Sulfolan gelöst und 16 h bei 80 °C unter Stickstoff gerührt. Der beim Verdünnen mit 1,5 l Aceton ausgefallene Niederschlag wurde abfiltriert, mit Aceton gewaschen und aus Methanol/Aceton umkristallisiert.

Ausbeute: 5,9 g (33%); Fp.: 245 °C; reine iso-Form.

Beispiel 21

$N_b$-(2-Hydroxy-2-phenyl-äthyl)-10-brom-isoajmalinium-hydrogentartrat:

10 g Bromisoajmalin und 3,3 ml Epoxystyrol wurden in 75 ml Äthanol 7 h bei 75 °C gerührt. Dann fügte man erneut 1 ml Epoxystyrol zu und rührte noch 13 h bei 75 °C. Die Reaktionsmischung wurde im Vakuum zur Trockene eingeengt, in Methanol aufgenommen, mit einer Lösung von 3,7 g L(+)-Weinsäure in wenig Methanol versetzt und das Hydrogentartrat durch Eintropfen in 1 l Essigester gefällt.

Ausbeute: 10,1 g (61%); Fp.: 148–152 °C.

Beispiel 22

$N_b$-[2-Hydroxy-3-(1-piperidinyl)-propyl]-10-brom-isoajmalinium-hydrogentartrat:

10 g Bromisoajmalin und 3,7 g 3-Piperidinopropylenoxid wurden 8 h in 75 ml Äthanol bei 75°C gerührt. Nachdem man nochmals 1 g 3-Piperidinopropylenoxid zugefügt hatte, rührte man weitere 8 h bei 75°C und destillierte dann das Lösungsmittel im Vakuum ab. Der Rückstand wurde in Aceton aufgenommen und in eine Lösung von 3,9 g L(+)-Weinsäure in 250 ml Aceton getropft. Das ausgefallene Hydrogentartrat wurde abgesaugt, die Aldehydbase freigesetzt und erneut mit 2,34 g L(+)-Weinsäure das Hydrogentartrat gefällt.

Ausbeute: 10 g (58%); Fp.: 130–135°C; n:iso ca. 2:3.

Beispiel 23

Tabletten, enthaltend $N_b$-Propyl-10-brom-isoajmalinium-hydrogentartrat.

Zusammensetzung

| | |
|---|---|
| Wirkstoff | 15 Teile |
| Lactose | 30 Teile |
| Maisstärke | 55 Teile |
| Gelatine | 1 Teil |
| Aerosil 200®[1] | 2 Teile |
| Hydriertes Rizinusöl | 2 Teile |
| Total | 105 Teile |

[1] Hochdisperses Siliziumdioxid mit 200 qm/g, Warenzeichen der Firma Degussa

Herstellungsvorschrift

Der Wirkstoff wird mit Lactose und Maisstärke vermischt. Die entstandene Mischung wird mit einer 15%igen wässrigen Lösung der Gelatine durchfeuchtet und granuliert. Die feuchte Masse wird durch ein 1,6-mm-Sieb passiert, bei 35°C auf Horden getrocknet und anschliessend durch ein 1,0-mm-Sieb passiert. Nach dem Vermischen des Granulates mit Aerosil 200® und gepulvertem hydrierten Rizinusöl werden damit Tabletten von 105 mg gepresst, so dass jede Tablette 15 mg Wirkstoff enthält.

Beispiel 24

Kapseln, enthaltend $N_b$-Propyl-10-brom-isoajmalinium-hydrogentartrat.

Zusammensetzung

| | |
|---|---|
| Wirksstoff | 15 Teile |
| Lactose | 55 Teile |
| Lösliche Stärke | 3 Teile |
| Aerosil 200®[1] | 2 Teile |
| Hydriertes Rizinusöl | 2 Teile |
| Total | 127 Teile |

[1] siehe Beispiel 23

Herstellungsvorschrift

Der Wirkstoff wird mit Lactose und Maisstärke vermischt. Die entstandene Mischung wird mit einer 15%igen wässrigen Lösung der löslichen Stärke durchfeuchtet und granuliert. Die feuchte Masse wird durch ein 1,6-mm-Sieb passiert, bei 35°C auf Horden getrocknet und anschliessend durch ein 1,0-mm-Sieb passiert. Nach dem Vermischen des Granulates mit Aerosil 200® und gepulvertem hydriertem Rizinusöl werden jeweils 127 mg der Mischung automatisch in Hartgelatinekapseln der Grösse 4 abgefüllt, so dass in jeder Kapsel 15 mg Wirkstoff enthalten sind.

Beispiel 25

Tabletten, enthaltend $N_b$-[2-(4-Morpholinyl)-äthyl]-10-brom-isoajmalinium-chlorid.

Zusammensetzung

| | |
|---|---|
| Wirkstoff | 15 Teile |
| Lactose | 60 Teile |
| Methylcellulose 2000cP | 10 Teile |
| Maisstärke | 15 Teile |
| Aerosil 200®[1] | 0,5 Teile |
| Magnesiumstearat | 0,5 Teile |
| Total | 101 Teile |

[1] siehe Beispiel 23

Herstellungsvorschrift

Der Wirkstoff wird zunächst mit Lactose, Methylcellulose und Maisstärke vermischt und dann mit den durch ein 0,2-mm-Sieb passierten Pulvern Aerosil 200® und Magnesiumstearat versetzt. Nach gründlicher Vermischung aller Bestandteile wird die Mischung auf dem Rundläufer zu Tabletten von 101 mg Gewicht verpresst, so dass jede Tablette 15 mg des Wirkstoffes enthält.

Beispiel 26

Kapseln, enthaltend $N_b$-[2-(4-Morpholinyl)-äthyl]-10-brom-isoajmalinium-chlorid.

Zusammensetzung

| | |
|---|---|
| Wirkstoff | 15 Teile |
| Lactose D 20 | 70 Teile |
| Mikrokristalline Cellulose | 30 Teile |
| Magnesiumstearat | 1 Teil |
| Total | 116 Teile |

Herstellungsvorschrift

Der Wirkstoff wird mit Lactose, mikrokristalliner Cellulose und Magnesiumstearat gründlich vermischt. Diese Pulvermischung wird automatisch so in Hartgelatinekapseln der Grösse 4 abgefüllt, dass jede Kapsel 15 mg Wirkstoff enthält.

Beispiel 27

a) $N_b$-n-Butyl-10-bromajmalinium-jodid:

10 g Bromajmalin werden in 250 ml Acetonitril gelöst, mit 8 ml 1-Jodbutan versetzt und 8 h unter Rückfluss erhitzt. Der ausgefallene Niederschlag wird abfiltriert und mit Methylenchlorid gewaschen.

Ausbeute: 12,5 g (86%); Fp.: 267–268°C; n:iso = 9:1.

b) $N_b$-n-Butyl-10-bromajmalinium-hydrogentartrat:

11,8 g $N_b$-n-Butyl-10-bromajmalinium-jodid werden mit kalter Sodalösung versetzt und erschöpfend mit Essigsäureäthylester extrahiert. Die getrocknete Essigesterlösung wird eingedampft, mit Methanol aufgenommen, mit 3 g L(+)-Weinsäure versetzt und etwas eingeengt. Das ausgefallene Hydrogentartrat wird abfiltriert.

Ausbeute: 9,3 g (76%); Fp.: 151 °C; n:iso = 9:1.

Beispiel 28

$N_b$-(Cyclopropylmethyl)-10-bromajmalinium-chlorid:

12 g Isoajmalin und 10 g Chlormethylcyclopropan werden in 100 ml Sulfolan unter Stickstoff 36 h auf 100 °C erhitzt. Nach dem Abkühlen wird mit 300 ml Aceton versetzt und die ausgefallenen Kristalle abfiltriert. Diese können aus Methanol umkristallisiert werden.

Ausbeute: 7,5 g (51%); Fp.: 268–273 °C; n:iso = 1:1.

Beispiel 29

a) $N_b$-(2-Phenyläthyl)-10-bromisoajmalinium-bromid:

8,5 g 10-Bromajamlin werden in 50 ml Äthanol aufgeschlämmt und mit 3,2 ml Phenyläthylbromid 44 h unter Rückfluss erhitzt. Das ausgefallene $N_b$-(2-Phenyläthyl)-10-bromisoajmalinium-bromid wird abfiltriert und kann zur weiteren Reinigung aus Äthanol umkristallisiert werden.

Ausbeute: 8,1 g (65%); Fp.: 210–212 °C; iso-Form.

b) $N_b$-(2-Phenyläthyl)-10-bromisoajmalinium-hydrogentartrat:

Die Darstellung erfolgt nach Variante B.

Ausbeute: 98%; Fp.: 155–160 °C; iso-Form.

**Patentansprüche für die Vertragsstaaten: BE, CH, DE, FR, GB, IT, LI, LU, NL, SE**

1. $N_b$-quartäre 10-Bromajmalin- und 10-Bromisoajmalin-Derivate der allgemeinen Formel I

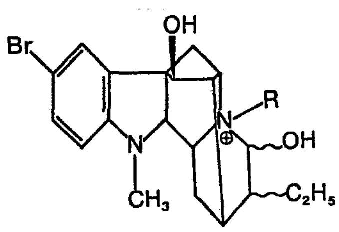

$A^{\ominus}$ (I)

in der

A das Anion einer anorganischen oder organischen Säure und

R ein bis zu 10 Kohlenstoffatome und gegebenenfalls Halogen oder Sauerstoff oder Stickstoff oder Sauerstoff und Stickstoff enthaltender Rest ist,

wobei

a) R ein Rest der allgemeinen Formel IIa

$$-(CH_2)_n-\underset{\displaystyle Y}{\underset{|}{C}}H-CH_2-X \qquad (IIa)$$

oder

b) R ein Rest der allgemeinen Formel IIb

$$-CH_2-\underset{\displaystyle OH}{\underset{|}{C}}H-CH_2-X \qquad (IIb)$$

ist, in denen

n den Wert 0 der 1 annehmen kann,

X Wasserstoff, gerad- oder verzweigtkettiges Alkyl, gegebenenfalls substituiertes Phenyl, Hydroxy, Dialkylamino, Pyrrolidino, Piperidino oder Morpholino und

Y Wasserstoff oder Methyl bedeuten

oder

c) R ein Methyl-, Allyl-, Cyclopropylmethyl-, Benzyl-, 4-Fluorbenzyl-, 4-Methoxybenzyl- oder 2-Hydroxy-2-phenyl-äthyl-Rest ist.

2. Derivate nach Anspruch 1, dadurch gekennzeichnet, dass R ein Äthyl-, Propyl-, Butyl-, 3-Methylbutyl-, Hexyl-, Decyl-, 2-Hydroxy-äthyl-, 2-Diäthylamino-äthyl-, 3-Diäthylamino-propyl-, 2-(1-Pyrrolidinyl)-äthyl-, 2-(1-Piperidinyl)-äthyl, 2-(4-Morpholinyl)-äthyl- oder 2-Hydroxy-3-(1-piperidinyl)-proypl-Rest ist.

3. Derivate nach Anspruch 1 oder 2, dadurch gekennzeichnet, dass A das Anion einer pharmakologisch annehmbaren Säure ist.

4. Derivate nach Anspruch 3, dadurch gekennzeichnet, dass A das Anion der Weinsäure ist.

5. Ring-offene Aldehydbasen der Formel IV

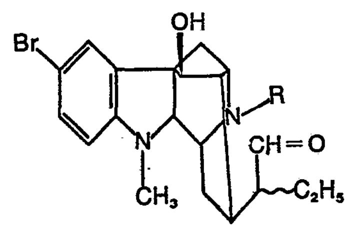

(IV)

in der R die im Anspruch 1 oder 2 genannte Bedeutung hat.

6. Verfahren zur Herstellung der Derivate nach einem der Ansprüche 1 bis 4, wobei R die in Anspruch 1 für die Alternativen a) und b) genannte Bedeutung hat, dadurch gekennzeichnet, dass man 10-Bromajmalin der Formel IIIn

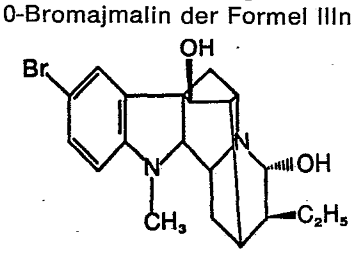

(III–n)

oder 10-Bromisoajmalin der Formel IIIi

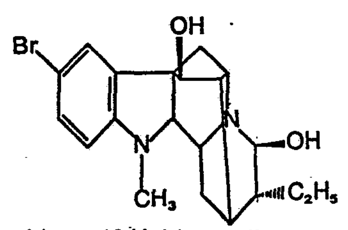

(III–i)

mit einer bis zu 10 Kohlenstoffatome enthaltenden Verbindung der Formeln Va oder Vb

$$Z-(CH_2)_n-\underset{\displaystyle Y}{\underset{|}{C}}H-CH_2-X \qquad (Va)$$

$$Z-CH_2-\underset{\displaystyle OH}{\underset{|}{C}}H-CH_2-X \qquad (Vb)$$

in denen, n, X und Y die an Anspruch 1 genannte Bedeutung haben und Z Chlor, Brom, Jod oder die Tosylgruppe bedeutet, umsetzt und gewünschtenfalls anschliessend die erhaltenen quartären Salze durch Behandeln mit Alkalien in die ring-offenen Aldehydbasen der Formel IV, in der R die vorstehend genannte Bedeutung hat, umwandelt und diese durch Umsetzen mit einer Säure HA, in der A die in einem der Ansprüche 1, 3 oder 4 genannte Bedeutung hat, in das quartäre Derivat der Formel I, in der R die vorstehend und A die in einem der Ansprüche 1, 3 oder 4 genannte Bedeutung haben, überführt.

7. Verfahren zur Herstellung der Derivate nach einem der Ansprüche 1 bis 4, wobei R die in Anspruch 1 für die Alternative b) genannte Bedeutung hat, dadurch gekennzeichnet, dass man 10-Bromajmalin der Formel IIIn oder 10-Bromisoajmalin der Formel IIIi mit einer bis zu 10 Kohlenstoffatome enthaltenden Verbindung der Formel Vb′

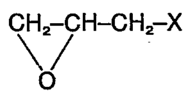

(Vb′)

in der X die im Anspruch 1 genannte Bedeutung hat, umsetzt und anschliessend die erhaltene ring-offene Aldehydbase der Formel IV, in der R die vorstehend genannte Bedeutung hat, durch Umsetzen mit einer Säure HA, in der A die in einem der Ansprüche 1, 3 oder 4 genannte Bedeutung hat, in das quartäre Derivat der Formel I, in der R die vorstehend und A die in einem der Ansprüche 1, 3 oder 4 genannte Bedeutung haben, überführt.

8. Verfahren zur Herstellung der Derivate nach einem der Ansprüche 1 bis 4, wobei R die in Anspruch 1 für die Alternative c) genannte Bedeutung, ausgenommen die Bedeutung 2-Hydroxy-2-phenyl-äthyl, hat, dadurch gekennzeichnet, dass man 10-Bromajmalin der Formel IIIn oder 10-Bromisoajmalin der Formel IIIi mit Methyl-, Allyl-, Cyclopropylmethyl-, Benzyl-, 4-Fluorbenzyl-, 4-Methoxybenzyl-Chlorid, Bromid, Jodid oder Tosylat umsetzt und gewünschtenfalls anschliessend die erhaltenen quartären Salze durch Behandeln mit Alkalien in die ringoffenen Aldehydbasen der Formel IV, in der R die vorstehend genannte Bedeutung hat, umwandelt und diese durch Umsetzen mit einer Säure HA, in der A die in einem der Ansprüche 1, 3 oder 4 genannte Bedeutung hat, in das quartäre Derivat der Formel I, in der R die vorstehend und A die in einem der Ansprüche 1, 3 oder 4 genannte Bedeutung haben, überführt.

9. Verfahren zur Herstellung der Derivate nach einem der Ansprüche 1 bis 4, wobei R 2-Hydroxy-2-phenyl-äthyl bedeutet, dadurch gekennzeichnet, dass man 10-Bromajmalin der Formel IIIn oder 10-Bromisoajmalin der Formel IIIi mit Epoxystyrol umsetzt und anschliessend die erhaltene ring-offene Aldehydbase der Formel IV, in der R 2-Hydroxy-2-phenyl-äthyl bedeutet, durch Umsetzen mit einer Säure HA, in der A die in einem der Ansprüche 1, 3 oder 4 genannte Bedeutung hat, in das quartäre Derivat der Formel I, in der R 2-Hydroxy-2-phenyl-äthyl bedeutet und A die in einem der Ansprüche 1, 3 oder 4 genannte Bedeutung hat, überführt.

10. Arzneimittel, enthaltend ein Derivat gemäss Anspruch 3 oder 4.

**Patentansprüche für den Vertragsstaat: AT**

1. Verfahren zur Herstellung von $N_b$-quartären 10-Bromajmalin- und 10-Bromisoajmalin-Derivaten der allgemeinen Formel I

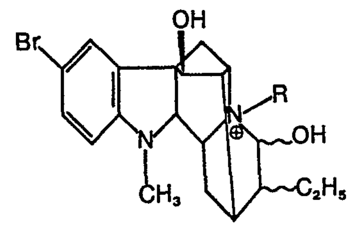

$A^{\ominus}$ (I)

in der

A das Anion einer anorganischen oder organischen Säure und

R ein bis zu 10 Kohlenstoffatome und gegebenenfalls Halogen oder Sauerstoff oder Stickstoff oder Sauerstoff und Stickstoff enthaltender Rest ist,

wobei

a) R ein Rest der allgemeinen Formel IIa

$-(CH_2)_n-CH-CH_2-X$ (IIa)
Y

oder

b) R ein Rest der allgemeinen Formel IIb

$-CH_2-CH-CH_2-X$ (IIb)
OH

ist, in denen

n den Wert 0 oder 1 annehmen kann,

X Wasserstoff, gerad- oder verzweigtkettiges Alkyl, gegebenenfalls substituiertes Phenyl, Hydroxy, Dialkylamino, Pyrrolidino, Piperidino oder Morpholino und

Y Wasserstoff oder Methyl bedeuten, dadurch gekennzeichnet, dass man 10-Bromajmalin der Formel IIIn

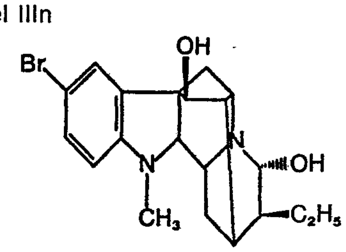

(III–n)

oder 10-Bromisoajmalin der Formel IIIi

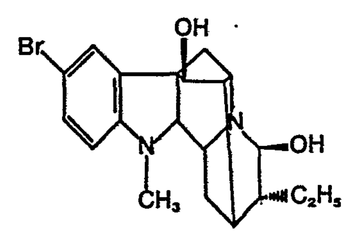

(III–i)

mit einer bis zu 10 Kohlenstoffatome enthaltenden Verbindung der Formeln Va oder Vb

$$Z-(CH_2)_n-\underset{\displaystyle Y}{\underset{|}{C}}H-CH_2-X \qquad (Va)$$

$$Z-CH_2-\underset{\displaystyle OH}{\underset{|}{C}}H-CH_2-X \qquad (Vb)$$

in denen n, X und Y die vorstehend genannte Bedeutung haben und Z Chlor, Brom, Jod oder die Tosylgruppe bedeutet, umsetzt und gewünschtenfalls anschliessend die erhaltenen quartären Salze durch Behandeln mit Alkalien in die ringoffenen Aldehydbasen der Formel IV

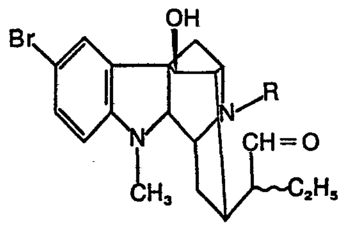

(IV)

in der R die vorstehend genannte Bedeutung hat, umwandelt und diese durch Umsetzen mit einer Säure HA, in der A die vorstehend genannte Bedeutung hat, in das quartäre Derivat der Formel I, in der R und A die vorstehend genannte Bedeutung haben, überführt.

2. Verfahren zur Herstellung von $N_b$-quartären 10-Bromajmalin- und 10-Bromisoajmalin-Derivaten der allgemeinen Formel I, in der

A das Anion einer anorganischen oder organischen Säure und

R ein bis zu 10 Kohlenstoffatome und gegebenenfalls Halogen oder Sauerstoff oder Stickstoff oder Sauerstoff und Stickstoff enthaltender Rest ist, wobei R ein Rest der allgemeinen Formel IIb

$$-CH_2-\underset{\displaystyle OH}{\underset{|}{C}}H-CH_2-X \qquad (IIb)$$

ist, in der X Wasserstoff, gerad- oder verzweigtkettiges Alkyl, gegebenenfalls substituiertes Phenyl, Hydroxy, Dialkylamino, Pyrrolidino, Piperidino oder Morpholino bedeutet, dadurch gekennzeichnet, dass man 10-Bromajmalin der Formel IIIn oder 10-Bromisoajmalin der Formel IIIi mit einer bis zu 10 Kohlenstoffatome enthaltenden Verbindung der Formel Vb'

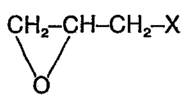

(Vb')

in der X die vorstehend genannte Bedeutung hat, umsetzt und anschliessend die erhaltene ringoffene Aldehydbase der Formel IV, in der R die vorstehend genannte Bedeutung hat, durch Umsetzen mit einer Säure HA, in der A die vorstehend genannte Bedeutung hat, in das quartäre Derivat der Formel I, in der R und A die vorstehend genannte Bedeutung haben, überführt.

3. Verfahren zur Herstellung von $N_b$-quartären 10-Bromajmalin- und 10-Bromisoajmalin-Derivaten der allgemeinen Formel I, in der A das Anion einer anorganischen oder organischen Säure und R ein Methyl-, Allyl-, Cyclopropylmethyl-, Benzyl-, 4-Fluorbenzyl- oder 4-Methoxybenzyl-Rest ist, dadurch gekennzeichnet, dass man 10-Bromajmalin der Formel IIIn oder 10-Bromisoajmalin der Formel IIIi mit einem Methyl-, Allyl-, Cyclopropylmethyl-, Benzyl-, 4-Fluorbenzyl-, 4-Methoxybenzyl-Chlorid, Bromid, Jodid oder Tosylat umsetzt und gewünschtenfalls anschliessend die erhaltenen quartären Salze durch Behandeln mit Alkalien in die ringoffenen Aldehydbasen der Formel IV, in der R die vorstehend genannte Bedeutung hat, umwandelt und diese durch Umsetzen mit einer Säure HA, in der A die vorstehend genannte Bedeutung hat, in das quartäre Derivat der Formel I, in der R und A die vorstehend genannte Bedeutung haben, überführt.

4. Verfahren zur Herstellung von $N_b$-quartären 10-Bromajmalin- und 10-Bromisoajmalin-Derivaten der allgemeinen Formel I, in der A das Anion einer anorganischen oder organischen Säure und R ein 2-Hydroxy-2-phenyl-äthyl-Rest ist, dadurch gekennzeichnet, dass man 10-Bromajmalin der Formel IIIn oder 10-Bromisoajmalin der Formel IIIi mit Epoxystyrol umsetzt und anschliessend die erhaltene ringoffene Aldehydbase der Formel IV, in der R die vorstehend genannte Bedeutung hat, durch Umsetzen mit einer Säure HA, in der A die vorstehend genannte Bedeutung hat, in das quartäre Derivat der Formel I, in der R und A die vorstehend genannte Bedeutung haben, überführt.

**Claims for the designated states BE, CH, DE, FR, GB, IT, LI, LU, NL, SE**

1. $N_b$-quaternary 10-bromoajmaline derivatives and 10-bromoisoajmaline derivatives of the general formula I

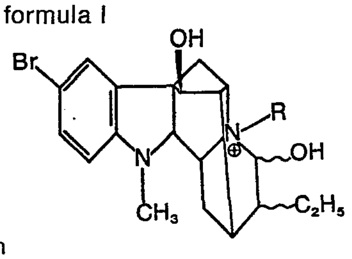

$A^{\ominus}$ (I)

in which

A is the anion of an inorganic or organic acid and

R is a radical containing up to 10 carbon atoms and optionally containing halogen or oxygen or nitrogen or oxygen and nitrogen,

where

a) R is a radical of the general formula IIa

$$-(CH_2)_n-\underset{\displaystyle Y}{\underset{|}{C}}H-CH_2-X \qquad (IIa)$$

or

b) R is a racidal of the general formula IIb

$$-CH_2-\underset{\displaystyle OH}{\underset{|}{C}}H-CH_2-X \qquad (IIb)$$

in which

n can assume the value 0 or 1,

X signifies hydrogen, straight-chain or branched alkyl, optionally substituted phenyl, hydroxy, dialkylamino, pyrrolidino, piperidino or morpholino and

Y signifies hydrogen or methyl,

or

c) R is a methyl-, allyl-, cyclopropylmethyl-, benzyl-, 4-fluorobenzyl-, 4-methoxybenzyl- or 2-hydroxy-2-phenyl-ethyl radical.

2. Derivatives according to Claim 1, characterized in that R is an ethyl, propyl, butyl, 3-methylbutyl, hexyl, decyl, 2-hydroxy-ethyl, 2-diethylamino-ethyl, 3-diethylamino-propyl, 2-(1-pyrrolidinyl)-ethyl, 2-(1-piperidinyl)-ethyl, 2-(4-morpholinyl)-ethyl or 2-hydroxy-3-(1-piperidinyl)-propyl radical.

3. Derivatives according to Claim 1 or 2, characterized in that A is the anion of a pharmacologically acceptable acid.

4. Derivatives according to Claim 3, characterized in that A is the anion of tartaric acid.

5. Ring-opened aldehyde bases of the formula IV

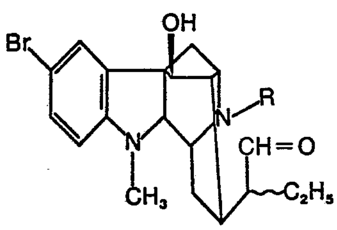

(IV)

in which R has the meaning indicated in Claim 1 or 2.

6. Process for the preparation of the derivatives according to one of Claims 1 to 4, in which R has the meaning indicated in Claim 1 for the alternatives a) and b), characterized in that 10-bromoajmaline of the formula IIIn

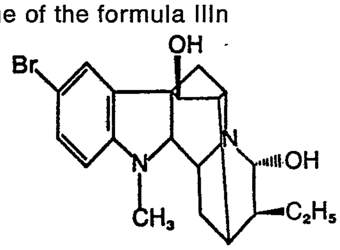

(III–n)

or 10-bromoisoajmaline of the formula IIIi

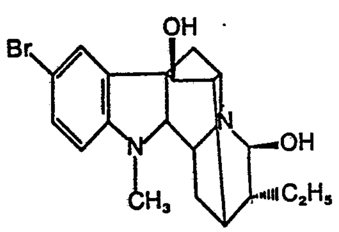

(III–i)

is reacted with a compound, containing up to 10 carbon atoms, of the formulae Va or Vb

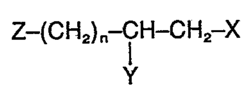

(Va)

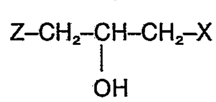

(Vb)

in which n, X and Y have the meaning indicated in Claim 1 and Z signifies chlorine, bromine, iodine or the tosyl group, and optionally subsequently the obtained quaternary salts are converted, through treatment with alkalis, into the ringopened aldehyde bases of the formula IV, in which R has the above-mentioned meaning, and these are converted into the quaternary derivative of formula I, in which R has the above-mentioned meaning and A has the meaning indicated in one of Claims 1, 3 or 4, by reaction with an acid HA, in which A has the meaning indicated in one of Claims 1, 3 or 4.

7. Process for the preparation of the derivatives according to one of Claims 1 to 4, in which R has the meaning indicated in Claim 1 for alternative b), characterized in that 10-bromoajmaline of the formula IIIn or 10-bromoisoajmaline of the formula IIIi is reacted with a compound, containing up to 10 carbon atoms, of the formula Vb′

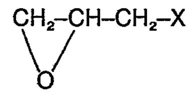

(Vb′)

in which X has the meaning indicated in Claim 1, and subsequently the obtained ring-opened aldehyde base of the formula IV, in which R has the above-mentioned meaning, is converted into the quaternary derivative of the formula I, in which R has the above-mentioned meaning and A has the meaning indicated in one of Claims 1, 3 or 4, by reaction with an acid HA, in which A has the meaning indicated in one of Claims 1, 3 or 4.

8. Process for the preparation of the derivatives according to one of Claims 1 to 4, in which R has the meaning indicated in Claim 1 for alternative c), except for the meaning 2-hydroxy-2-phenyl-ethyl, characterized in that 10-bromoajmaline of the formula IIIn or 10-bromoisoajmaline of the formula IIIi is reacted with methyl-, allyl-, cyclopropylmethyl-, benzyl-, 4-fluorobenzyl-, 4-methoxybenzyl-chloride, bromide, iodide or tosylate and optionally subsequently the obtained quaternary salts are coverted, by treatment with alkalis, into the ring-opened aldehyde bases of formula IV, in which R has the above-mentioned meaning, and these are converted into the quaternary derivative of formula I, in which R has the meaning indicated above and A has the meaning indicated in Claims 1, 3 or 4, by reaction with an acid HA, in which A has the meaning indicated in one of Claims 1, 3 or 4.

9. Process for the preparation of the derivatives according to one of Claims 1 to 4, in which R signifies 2-hydroxy-2-phenyl-ethyl, characterized in that 10-bromoajmaline of the formula IIIn or 10-bromoisoajmaline of the formula IIIi is reacted with epoxystyrene and subsequently the obtained ring-opened aldehyde base of formula IV, in which R signifies 2-hydroxy-2-phenyl-ethyl, is converted into the quaternary derivative of formula I, in which R signifies 2-hydroxy-2-phenyl-ethyl and A has the meaning indicated in one of Claims 1, 3 or 4, by reaction with an acid HA, in which A has the meaning indicated in one of Claims 1, 3 or 4.

10. Medicaments containing a derivative according to Claim 3 or 4.

**Claims for the designated state AT**

1. Process for the preparation of $N_b$-quaternary 10-bromoajmaline derivatives and 10-bromoisoajmaline derivates fo the general formula I

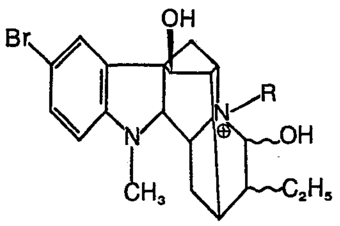

$A^{\ominus}$ (I)

in which

A is the anion of an inorganic or organic acid and

R is a radical containing up to 10 carbon atoms and optionally containing halogen or oxygen or nitrogen or oxygen and nitrogen,

where

a) R is a radical of the general formula IIa

$$-(CH_2)_n-\underset{\displaystyle Y}{\underset{|}{C}}H-CH_2-X \qquad \text{(IIa)}$$

or

b) R is a radical of the general formula IIb

$$-CH_2-\underset{\displaystyle OH}{\underset{|}{C}}H-CH_2-X \qquad \text{(IIb)}$$

in which

n can assume the value 0 or 1,

X signifies hydrogen, straight-chain or branched alkyl, optionally substituted phenyl, hydroxy, dialkylamino, pyrrolidino, piperidino or morpholino and

Y signifies hydrogen or methyl, characterized in that 10-bromoajmaline of the formula IIIn

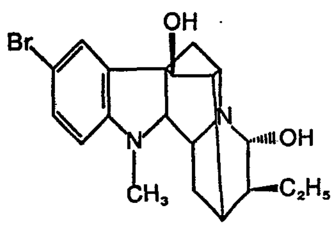

(III–n)

or 10-bromoisoajmaline of the formula IIIi

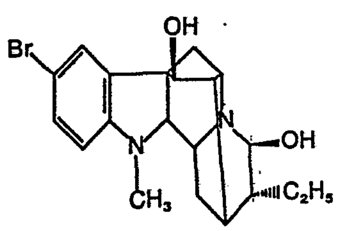

(III–i)

is reacted with a compound, containing up to 10 carbon atoms, of the formulae Va or Vb

$$Z-(CH_2)_n-\underset{\displaystyle Y}{\underset{|}{C}}H-CH_2-X \qquad \text{(Va)}$$

$$Z-CH_2-\underset{\displaystyle OH}{\underset{|}{C}}H-CH_2-X \qquad \text{(Vb)}$$

in which n, X and Y have the above-mentioned meaning and Z signifies chlorine, bromine, iodine or the tosyl group, and optionally subsequently the obtained quaternary salts are converted, through treatment with alkalis, into the ring-opened aldehyde bases of the formula IV

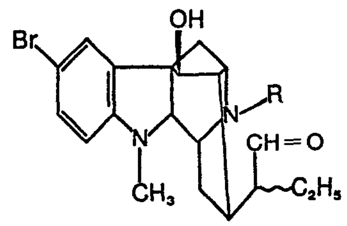

(IV)

in which R has the above-mentioned meaning, and these are converted into the quaternary derivative of formula I, in which R and A have the above-mentioned meaning, through reaction with an acid HA, in which A has the above-mentioned meaning.

2. Process for the preparation of $N_b$-quaternary 10-bromoajmaline derivatives and 10-bromoisoajmaline derivatives of the general formula I, in which

A is the anion of an inorganic or organic acid and

R is a radical containing up to 10 carbon atoms and optionally containing halogen or oxygen or nitrogen or oxygen and nitrogen, where R is a radical of the general formula IIb

$$-CH_2-\underset{\displaystyle OH}{\underset{|}{C}}H-CH_2-X \qquad \text{(IIb)}$$

in which X signifies hydrogen, straight-chain or branched alkyl, optionally substituted phenyl, hydroxy, dialkylamino, pyrrolidino, piperidino or morpholino, characterized in that 10-bromoajmaline of the formula IIIn or 10-bromoisoajmaline of the formula IIIi is reacted with a compound, containing up to 10 carbon atoms, of the formula Vb′

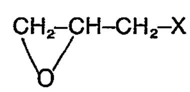

(Vb′)

in which X has the above-mentioned meaning, and subsequently the obtained ring-opened aldehyde base of the formula IV, in which R has the above-mentioned meaning, is converted into the quaternary derivative of the formula I, in which R and A have the above-mentioned meaning, by reaction with an acid HA, in which A has the above-mentioned meaning.

3. Process for the preparation of $N_b$-quaternary 10-bromoajmaline derivates and 10-bromoisoajmaline derivatives of the general formula I, in

which A is the anion of an inorganic or organic acid and

R is a methyl-, allyl-, cyclopropylmethyl-, benzyl-, 4-fluorobenzyl- or 4-methoxybenzyl-radical characterized in that 10-bromoajmaline of the formula IIIn of 10-bromoisoajmaline of the formula IIIi is reacted with a methyl-, allyl-, cyclopropylmethyl-, benzyl-, 4-fluorobenzyl-, 4-methoxybenzyl-chloride, bromide, iodide or tosylate and optionally, subsequently, the obtained quaternary salts are converted, by treatment with alkalis, into the ring-opened aldehyde bases of formula IV, in which R has the above-mentioned meaning, and these are converted into the quaternary derivative of formula I, in which R and A have the above-mentioned meaning, by reaction with an acid HA, in which A has the above-mentioned meaning.

4. Process for the preparation of $N_b$-quaternary 10-bromoajmaline derivatives and 10-bromoisoajmaline derivatives of the general formula I, in which A is the anion of an inorganic or organic acid and R is a 2-hydroxy-2-phenyl-ethyl radical, characterized in that 10-bromoajmaline of the formula IIIn or 1o-bromoisoajmaline of the formula IIIi is reacted with epoxystyrene and subsequently the obtained ring-opened aldehyde base for formula IV, in which R has the above-mentioned meaning, is converted into the quaternary derivative of formula I, in which R and A have the above-mentioned meaning, by reaction with an acid HA, in which A has the above-mentioned meaning.

**Revendications pour les Etats contractants: BE, CH, DE, FR, GB, IT, LI, LU NL, SE**

1. Dérivés $N_b$-quaternaires de 10-bromo-ajmaline et de 10-bromoisoajmaline, de formule générale I

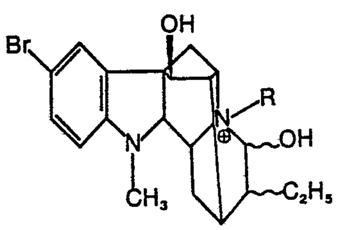

$A^{\ominus}$ (I)

dans laquelle A est l'anion d'un acide inorganique ou organique et R est un radical contenant jusqu'à 10 atomes de carbone et éventuellement un halogène ou de l'oxygène ou de l'azote ou de l'oxygène et de l'azote,

où

a) R est un radical de formule générale IIa

$-(CH_2)_n-CH(Y)-CH_2-X$ (IIa)

ou

b) R est un radical de formule générale IIb

$-CH_2-CH(OH)-CH_2-X$ (IIb)

dans lesquelles n peut prendre la valeur 0 ou 1; X est l'hydrogène, un radical alkyle à chaîne droite ou ramifiée, phényle éventuellement substitué, hydroxy, dialkylamino, pyrrolidino, pipéridino ou morpholino; et Y est l'hydrogène ou le radical méthyle;

ou bien

c) R est un radical méthyle, allyle, cyclopropylméthyle, benzyle, 4-fluorobenzyle, 4-méthoxybenzyle ou 2-hydroxy-2-phényl-éthyle.

2. Dérivés selon la revendication 1, caractérisés en ce que R est un radical éthyle, propyle, butyle, 3-méthylbutyle, hexyle, décyle, 2-hydroxy-éthyle, 2-diéthylaminoéthyle, 3-diéthylamino-propyle, 2-(1-pyrrolidinyl)-éthyle, 2-(1-pipéridinyl)-éthyle, 2-(4-morpholinyl)-éthyle ou 2-hydroxy-3-(1-pipéridinyl)-propyle.

3. Dérivés selon la revendication 1 ou 2, caractérisés en ce que A est l'anion d'un acide pharmacologiquement acceptable.

4. Dérivés selon la revendication 3, caractérisés en ce que A est l'anion de l'acide tartrique.

5. Aldéhydes-bases à cycle ouvert, de formule IV

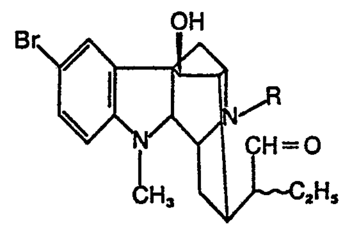

(IV)

dans laquelle R a la signification donnée dans la revendication 1 ou 2.

6. Procédé pour la préparation des dérivés selon l'une des revendications 1 à 4, où R a la signification donnée dans la revendication 1 aux points a) et b), caractérisé en ce que l'on fait réagir la 10-bromo-ajmaline de formule IIIn

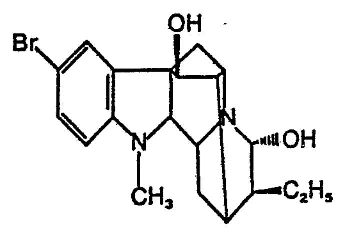

(III–n)

ou la 10-bromo-isoajmaline de formule IIIi

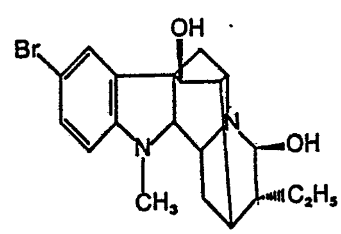

(III–i)

sur un composé contenant jusqu'à 10 atomes de carbone, de formules Va ou Vb

$Z-(CH_2)_n-CH(Y)-CH_2-X$ (Va)

$Z-CH_2-CH(OH)-CH_2-X$ (Vb)

dans lesquelles n, X et Y ont la signification donnée dans la revendication 1 et Z est le chlore, le brome, l'iode ou le groupe tosyle, et que, si on le souhaite, on transforme ensuite les sels quaternaires obtenus par traitement avec des alcalis en les aldéhydes-bases à cycle ouvert de formule IV dans laquelle R a la signification donnée ci-dessus, et que l'on convertit ces dernières par réaction avec un acide HA, où A a la signification donnée dans l'une des revendications 1, 3 ou 4, en le dérivé quaternaire de formule I où R a la signification donnée ci-dessus et A la signification donnée dans l'une des revendications 1, 3 ou 4.

7. Procédé pour la préparation de dérivés selon l'une des revendications 1 à 4, où R a la signification donnée dans la revendication 1 au point b), caractérisé en ce qu'on fait réagir de la 10-bromo-ajmaline de formule IIIn ou de la 10-bromo-isoajmaline de formule IIIi sur un composé contenant jusqu'à 10 atomes de carbone, de formule Vb'

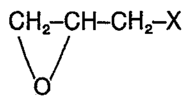

(Vb')

où X a la signification donnée dans la revendication 1, et qu'on convertit ensuite l'aldéhyde-base à cycle ouvert obtenue, de formule IV, dans laquelle R a la signification donnée ci-dessus, et par réaction sur un acide HA, où A a la signification donnée dans l'une des revendications 1, 3 ou 4, en le dérivé quaternaire de formule I où R a la signification donnée ci-dessus et A a la signification donnée dans l'une des revendications 1, 3 ou 4.

8. Procédé pour la préparation des dérivés selon l'une des revendications 1 à 4, où R a la signification donnée dans la revendication 1 au point c), à l'exception de la signification 2-hydroxy-2-phényl-éthyle, caractérisé en ce qu'on fait réagir de la 10-bromo-ajmaline de formule IIIn ou de la 10-bromo-isoajmaline de formule IIIi sur du chlorure, bromure, iodure ou tosylate de méthyle, allyle, cyclopropylméthyle, benzyle, 4-fluorobenzyle, 4-méthoxybenzyle, et que, si on le souhaite, on transforme ensuite les sels quaternaires obtenus, par traitement avec des alcalis, en les aldéhydes-bases à cycle ouvert de formule IV où R a la signification donnée ci-dessus, et qu'on convertit ces dernières, par réaction avec un acide HA où A a la signification donnée dans l'une des revendications 1, 3 ou 4, en le dérivé quaternaire de formule I où R a la signification donnée ci-dessus et A la signification donnée dans l'une des revendications 1, 3 ou 4.

9. Procédé pour la préparation des dérivés selon l'une des revendications 1 à 4, où R est le radical 2-hydroxy-2-phényl-éthyle, caractérisé en ce qu'on fait réagir de la 10-bromo-ajmaline de formule IIIn ou de la 10-bromo-isoajmaline de formule IIIi sur de l'époxystyrène, puis que l'on convertit l'aldéhyde-base à cycle ouvert obtenue de formule IV, où R est le radical 2-hydroxy-2-phényl-éthyle, par réaction avec un acide HA où A a la signification donnée dans l'une des revendications 1, 3 ou 4, en le dérivé quaternaire de formule I où R est le radical 2-hydroxy-2-phényl-éthyle et A a la signification donnée dans l'une des revendications 1, 3 ou 4.

10. Médicament contenant un dérivé selon la revendication 3 ou 4.

**Revendications pour l'Etat contractant AT**

1. Procédé pour la préparation de dérivés $N_b$-quaternaires de 10-bromo-ajmaline et de 10-bromo-isoajmaline de formule générale

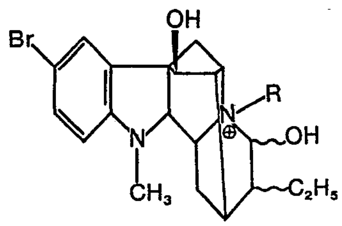

$A^{\ominus}$ (I)

où A est l'anion d'un acide inorganique ou organique et R est un radical contenant jusqu'à 10 atomes de carbone et éventuellement un halogène ou de l'oxygène ou de l'azote ou de l'oxygène et de l'azote,
où

a) R est un radical de formule générale IIa

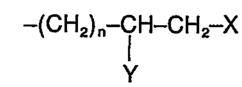

(IIa)

ou

b) R est un radical de formule générale IIb

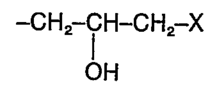

(IIb)

dans lesquelles n peut prendre la valeur 0 ou 1; X est l'hydrogène ou un radical alkyle à chaîne droite ou ramifiée, phényle éventuellement substitué, hydroxy, dialkylamino, pyrrolidino, pipéridino ou morpholino; et Y est l'hydrogène ou le radical méthyle; caractérisé en ce que l'on fait réagir de la 10-bromo-ajmaline de formule IIIn

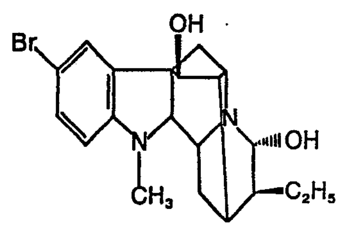

(III–n)

ou de la 10-bromo-isoajmaline de formule IIIi

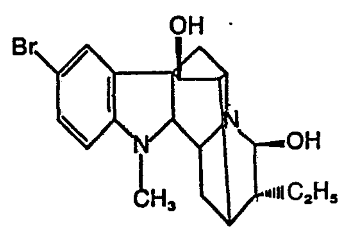

(III–i)

sur un composé contenant jusqu'à 10 atomes de carbone, de formule Va ou Vb,

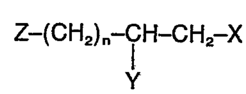

(Va)

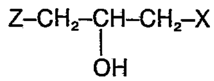 (Vb)

dans lesquelles n, X et Y ont la signification donnée ci-dessus et Z est le chlore, le brome, l'iode ou le groupe tosyle, et que, si on le souhaite, on transforme les sels quaternaires obtenus par traitement avec des alcalis, en les aldéhydes-bases à cycle ouvert de formule IV

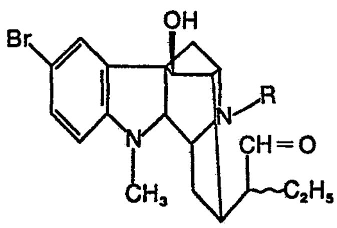 (IV)

dans laquelle R a la signification donnée ci-dessus, et qu'on convertit ces dernières par réaction sur un acide HA, où A a la signification donnée ci-dessus, en le dérivé quaternaire de formule I où R et A ont la signification donnée ci-dessus.

2. Procédé pour la préparation de dérivés $N_b$-quaternaires de 10-bromo-ajmaline et de 10-bromo-isoajmaline de formule générale I, dans laquelle A est l'anion d'un acide inorganique ou organique et R est un radical contenant jusqu'à 10 atomes de carbone et éventuellement un halogène ou de l'oxygène ou de l'azote ou de l'oxygène et de l'azote, où R est un radical de formule générale IIb

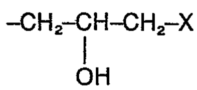 (IIb)

dans laquelle X est l'hydrogène, un radical alkyle à chaîne droite ou ramifiée, phényle éventuellement substitué, hydroxy, dialkylamino, pyrrolidino, pipéridino ou morpholino; caractérisé en ce qu'on fait réagir de la 10-bromo-ajmaline de formule IIIn ou de la 10-bromo-isoajmaline de formule IIIi sur un composé contenant jusqu'à 10 atomes de carbone, de formule Vb′

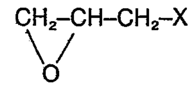 (Vb′)

dans laquelle X a la signification ci-dessus, puis qu'on convertit l'aldéhyde-base à cycle ouvert obtenue, de formule IV dans laquelle R a la signification donnée ci-dessus, par réaction avec un acide HA ou A a la signification donnée ci-dessus, en le dérivé quaternaire de formule I ou R et A ont la signification donnée ci-dessus.

3. Procédé pour la préparation de dérivés $N_b$-quaternaires de 10-bromo-ajmaline et de 10-bromo-isoajmaline de formule générale I dans laquelle A est l'anion d'un acide inorganique ou organique et R est un radical méthyle, allyle, cyclopropylméthyle, benzyle, 4-fluorobenzyle ou 4-méthoxybenzyle, caractérisé en ce qu'on fait réagir de la 10-bromo-ajmaline de formule IIIn ou de la 10-bromo-isoajmaline de formule IIIi sur un chlorure, bromure, iodure ou tosylate de méthyle, allyle, cyclopropylméthyle, benzyle, 4-fluorobenzyle, 4-méthoxybenzyle, et que, si on le souhaite, on transforme ensuite les sels quaternaires obtenus, par traitement avec des alcalis, en les aldéhydes-bases à cycle ouvert de formule IV dans laquelle R a la signification donnée ci-dessus, et qu'on transforme ces dernières, par réaction avec un acide HA où A a la signification donnée ci-dessus, en le dérivé quaternaire de formule I dans laquelle R et A ont la signification donnée ci-dessus.

4. Procédé pour la préparation de dérivés $N_b$-quaternaires de 10-bromo-ajmaline et de 10-bromo-isoajmaline de formule générale I, dans laquelle A est l'anion d'un acide inorganique ou organique et R est un radical 2-hydroxy-2-phényléthyle, caractérisé en ce qu'on fait réagir de la 10-bromo-ajmaline de formule IIIn ou de la 10-bromo-isoajmaline de formule IIIi sur de l'époxystyrène, puis que l'on transforme l'aldéhyde-base à cycle ouvert obtenue, de formule IV dans laquelle R a la signification donnée ci-dessus, par réaction avec un acide HA, où A a la signification donnée ci-dessus, en le dérivé quaternaire de formule I dans laquelle R et A ont la signification donnée ci-dessus.

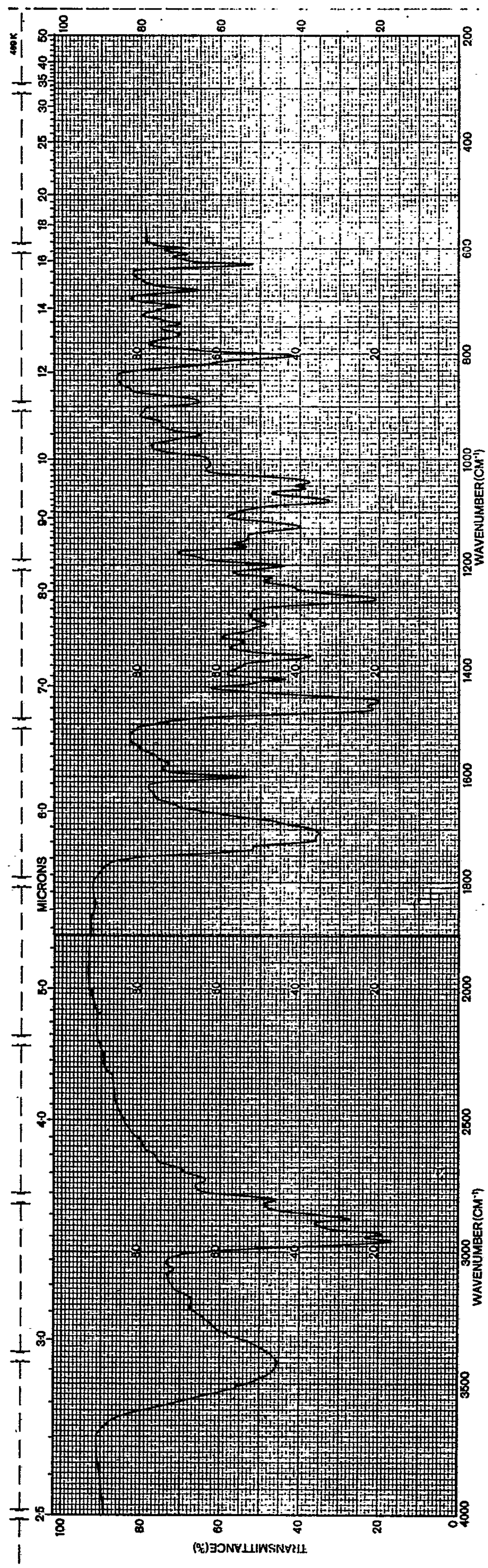


FIG.1

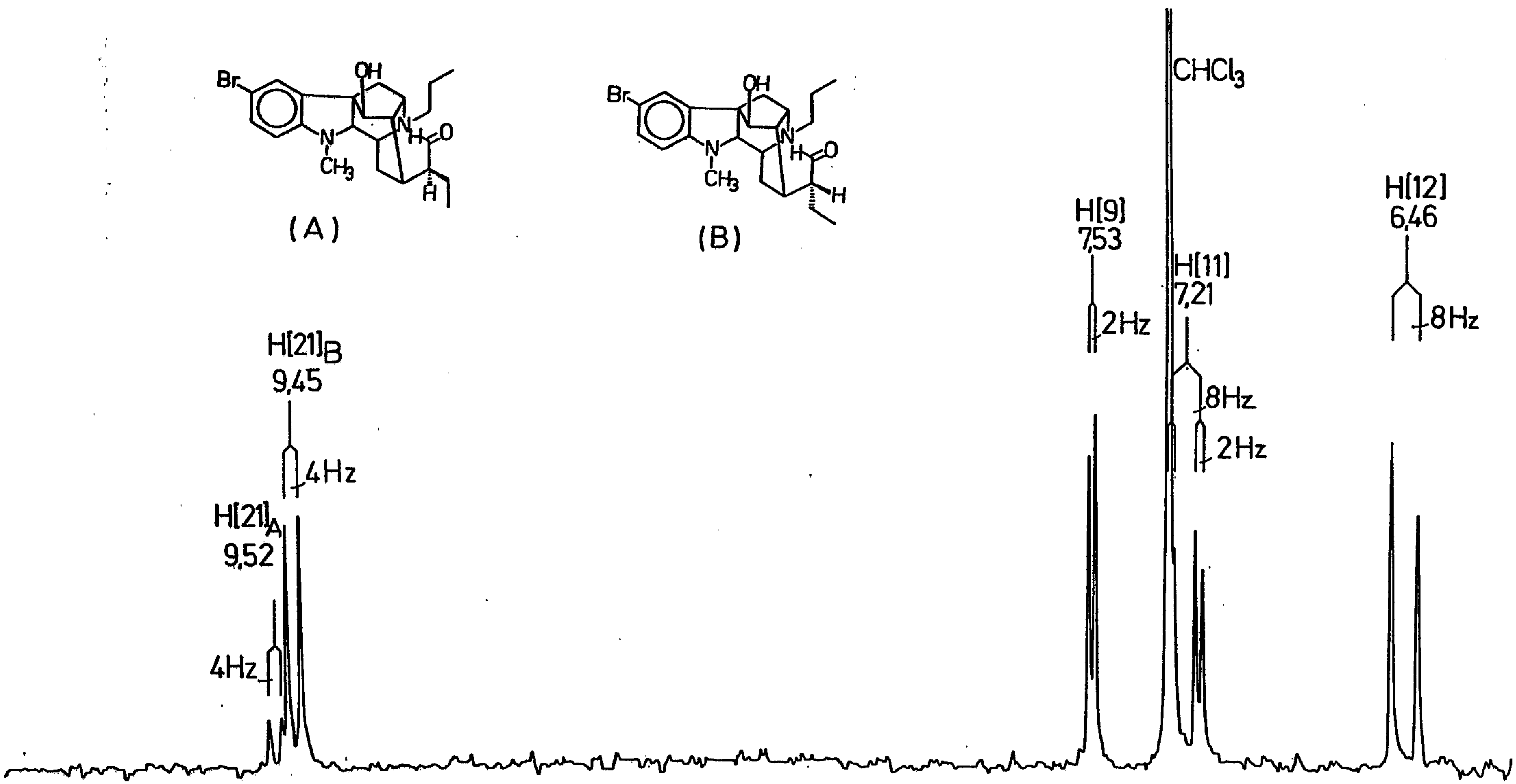

CHCl3
H[21]B
9,45
4Hz
H[21]A
9,52
4Hz
H[9]
7,53
2Hz
H[11]
7,21
8Hz
2Hz
H[12]
6,46
8Hz


(A)

(B)

FIG.2